(19) Europäisches Patentamt
European Patent Office
Office européen des brevets

(11) Veröffentlichungsnummer: **0 012 273**
**A1**

(12) # EUROPÄISCHE PATENTANMELDUNG

(21) Anmeldenummer: **79104736.8**

(22) Anmeldetag: **28.11.79**

(51) Int. Cl.³: **C 07 C 69/747**, C 07 C 69/767, C 07 C 67/14, A 01 N 53/00, C 07 D 307/28, A 01 N 43/08, A 01 N 37/10 // C07C43/295, C07C33/28

(30) Priorität: **02.12.78 DE 2852275**

(43) Veröffentlichungstag der Anmeldung: **25.06.80**
**Patentblatt 80/13**

(84) Benannte Vertragsstaaten: **AT BE CH DE FR GB IT NL**

(71) Anmelder: **BASF Aktiengesellschaft,**
**Carl-Bosch-Strasse 38, D-6700 Ludwigshafen (DE)**

(72) Erfinder: **Schwarz, Gerd-Ulrich, Dr. Dipl.-Chem.,**
**Danziger Strasse 10, D-6800 Mannheim 71 (DE)**
Erfinder: **Klehs, Karl, Dr. Dipl.-Chem.,**
**Sudetenstrasse 22, D-6840 Lampertheim (DE)**
Erfinder: **Adolphi, Heinrich, Dr. Dipl.-Bio, Kalmitweg 11,**
**D-6703 Limburgerhof (DE)**

(54) **Carbonsäureester, Verfahren zu ihrer Herstellung und ihre Verwendung zur Bekämpfung von Schädlingen.**

(57) Die vorliegende Erfindung betrifft neue Carbonsäureester der Formel

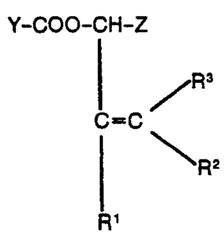

in der
$R^1$, $R^2$ und $R^3$ gleich oder verschieden sind und für Wasserstoff, Halogen, Alkyl oder Alkenyl mit bis zu 5 Kohlenstoffatomen,
Y für einen 3-(2,2-Dihalogenvinyl)-2,2-dimethyl-cyclopropylrest oder einen Rest der Formel

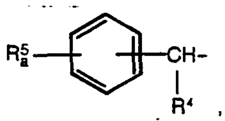

wobei $R^4$ unverzweigtes oder verzweigtes Alkyl, Alkenyl oder Alkinyl mit bis zu 4 Kohlenstoffatomen oder einen alicyclischen Rest mit 3 bis 7 Kohlenstoffatomen,
$R^5$ Halogen, Alkyl oder Alkoxy mit bis zu 5 Kohlenstoffatomen, Trihalogenmethyl, Cyano oder Nitro und
a 0 bis 3 bedeuten, und
Z für einen Rest der Formel

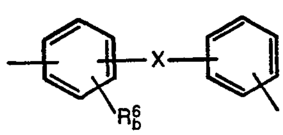

oder

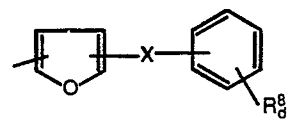

wobei X Sauerstoff, Schwefel oder $-CH_2-$,
$R^6$, $R^7$ und $R^8$ Halogen, Alkyl, Alkoxy oder Halogenalkyl mit bis zu 5 Kohlenstoffatomen und b, c und d 0 bis 3 bedeuten, stehen, ihre Herstellung und Verwendung zur Bekämpfung von Schädlingen.

EP 0 012 273 A1

Carbonsäureester, Verfahren zu ihrer Herstellung und ihre Verwendung zur Bekämpfung von Schädlingen

Gegenstand der Erfindung sind neue Carbonsäureester, ein Verfahren zu ihrer Herstellung und Schädlingsbekämpfungsmittel, die diese Ester als Wirkstoffe enthalten.

Es ist bekannt, daß 2,2,-Dimethyl-3-(2',2'-dimethylvinyl)--cyclopropancarbonsäureester, die als Alkoholkomponente einen in α-Stellung durch Alkenyl substituierten Furylmethylrest enthalten, insektizid wirksam sind (BE-PS 770 874). Weiterhin ist aus Agr. Biol.-Chem. 37, 2681 (1973) bekannt, daß der in α-Stellung am Phenoxybenzylrest durch Vinyl substituierte 2,2-Dimethyl-3-(2',2'--dimethylvinyl)-cyclopropancarbonsäure-(3"-phenoxybenzyl)--ester insektizid wirksam ist. Diese Verbindung ist jedoch insektizid weniger wirksam als der Ester, der als Alkoholkomponente den unsubstituierten 3-Phenoxy-benzylrest enthält (Agr. Biol.-Chem. 40, 247-249 (1976)).

Es wurde gefunden, daß Carbonsäureester der Formel I

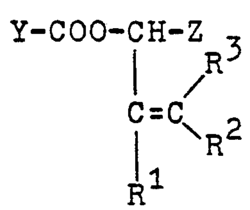

I,

in der
$R^1$, $R^2$ und $R^3$ gleich oder verschieden sind und für Wasserstoff, Halogen, Alkyl oder Alkenyl mit bis zu 5 Kohlenstoffatomen,
Y für einen 3-(2,2-Dihalogenvinyl)-2,2-dimethyl-cyclopropylrest oder einen Rest der Formel

H/Kl

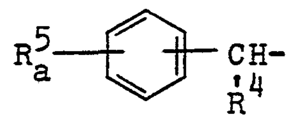
,

wobei $R^4$ unverzweigtes oder verzweigtes Alkyl, Alkenyl oder Alkinyl mit bis zu 4 Kohlenstoffatomen oder einen alicyclischen Rest mit 3 bis 7 Kohlenstoffatomen,
$R^5$ Halogen, Alkyl oder Alkoxy mit bis zu 5 Kohlenstoffatomen, Trihalogenmethyl, Cyano oder Nitro und a 0 bis 3 bedeuten, und
Z für einen Rest der Formel

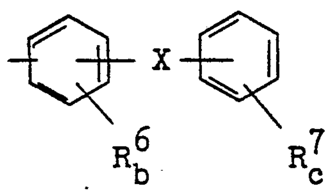
oder
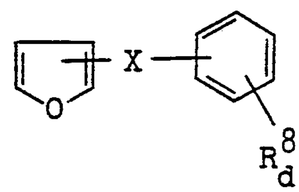

wobei X Sauerstoff, Schwefel oder $-CH_2-$,
$R^6$, $R^7$ und $R^8$ Halogen, Alkyl, Alkoxy oder Halogenalkyl mit bis zu 5 Kohlenstoffatomen und b, c und d 0 bis 3 bedeuten,
stehen,
sich sehr gut zur Bekämpfung von Schädlingen, insbesondere von Insekten und Spinnentieren, eignen.

In der Formel I steht Y für einen 3-(2,2-Dihalogenvinyl)-2,2-dimethyl-cyclopropylrest, beispielsweise für den 3-(2,2-Dichlorvinyl)-2,2-dimethyl-cyclopropyl-, den 3-(2,2-Dibromvinyl)-2,2-dimethylcyclopropyl- oder den 3-(2,2-Difluorvinyl)-2,2-dimethyl-cyclopropylrest, oder einen Rest der Formel

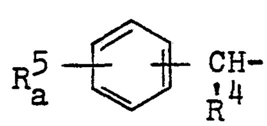

der bis zu drei gleiche oder verschiedene Substituenten $R^5$ am Phenylring tragen kann. Als Substituenten $R^5$ kommen dabei Halogen, beispielsweise Fluor, Chlor oder Brom, Trihalogenmethyl, beispielsweise Trifluormethyl, Trichlor-

methyl, Cyano, Nitro oder unverzweigte oder verzweigte Alkyl- oder Alkoxygruppen mit bis zu 5-Kohlenstoffatomen, beispielsweise Methyl, Methoxy, Äthyl, Äthoxy, n-Propyl, Isopropyl, tert.-Butyl, Isobutyl, Pentyl, Isopentyl, n-Propoxy, n-Butoxy, Isopropoxy, sec.-Butoxy, Isobutoxy, n-Pentoxy, Isopentoxy, in Betracht.

$R^4$ kann unverzweigtes oder verzweigtes Alkyl, Alkenyl oder Alkinyl mit bis zu 4 Kohlenstoffatomen, beispielsweise Methyl, Äthyl, Isopropyl, tert.-Butyl, Isobutyl, Allyl, Isopropenyl, Propargyl, oder einen alicyclischen Rest mit 3 bis 7 Kohlenstoffatomen, beispielsweise Cyclopropyl oder Cyclohexyl, bedeuten.

Z in Formel I steht für Reste der Formeln

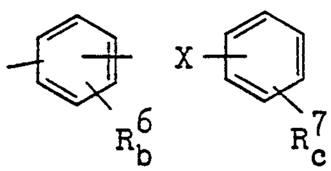

oder

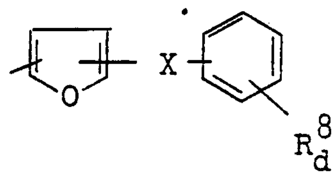

in denen X für Sauerstoff, Schwefel oder eine Methylengruppe steht, beispielsweise 3-Phenoxyphenyl, 5-Benzyl-furyl-(3), 5-Phenoxy-furyl-(3), 5-Phenoxy-furyl-(2), 3-Benzylphenyl, 3-Phenylthiophenyl, 5-Phenylthio-furyl-(3). Die Phenylringe in den Formeln der Reste für Z können gegebenenfalls jeweils bis zu drei gleiche oder verschiedene Substituenten tragen. Als Substituenten $R^6$, $R^7$ und $R^8$ kommen Halogen, beispielsweise Fluor, Chlor, Brom, unverzweigte oder verzweigte Alkyl-, Alkoxy- oder Halogenalkylgruppen mit bis zu 5 Kohlenstoffatomen, beispielsweise Methyl, Äthyl, Propyl, Isopropyl, n-Butyl, tert.-Butyl, Isobutyl, isomere Pentylreste, Methoxy, Äthoxy, n-Propoxy, Isopropoxy, isomere Butoxy- oder Pentoxyreste, Trifluormethyl, Trichlormethyl, in Betracht.

0012273

$R^1$, $R^2$ und $R^3$ in Formel I bedeuten unabhängig voneinander Wasserstoff, Halogen, wie Fluor, Chlor, Brom, unverzweigtes oder verzweigtes Alkyl oder Alkenyl mit bis zu 5 Kohlenstoffatomen, beispielsweise Methyl, Äthyl, n-Propyl, Isopropyl, Butyl, tert.-Butyl, n-Pentyl, Isopentyl, Vinyl, Allyl, n-Propenyl, Isopropenyl, n-Butenylreste, n-Pentenylreste, 1-Methyl-n-propenylreste, 1-Methyl-n-butenylreste.

Bevorzugte Verbindungen der Formel I sind solche, bei denen Y einen 3-(2,2-Dihalogenvinyl)-2,2-dimethyl-cyclopropylrest oder einen am Phenylring durch Fluor, Chlor, Brom oder Alkoxy mit bis zu 5 Kohlenstoffatomen einfach substituierten α-Isopropylbenzylrest, Z den 3-Phenoxy-phenylrest oder den 5-Benzyl-furyl-(3)-rest und $R^1$, $R^2$ und $R^3$ Wasserstoff oder Fluor bedeuten.

Beispiele für erfindungsgemäße Ester der Formel I sind 2,2-Dimethyl-3-(2',2'-dichlorvinyl)-cyclopropancarbonsäure-(3-phenoxy-α-vinyl-benzyl)-ester, 2,2-Dimethyl-3-(2',2'-dibromvinyl)-cyclopropylcarbonsäure-(3-phenoxy-α-vinyl-benzyl)-ester, 2,2-Dimethyl-3-(2',2'-difluorvinyl)-cyclopropancarbonsäure-(3-phenoxy-α-vinyl-benzyl)-ester, 2-(4-Chlorphenyl)-isovaleriansäure-(3-phenoxy-α-vinyl-benzyl)-ester, 2,2-Dimethyl-3-(2',2'-difluorvinyl)-cyclopropancarbonsäure-(5-benzyl-α-vinyl-furfuryl-3)-ester, 2,2-Dimethyl-3-(2',2'-difluorvinyl)-cyclopropancarbonsäure-[5-benzyl-α-(2-methyl-propen-1-yl)-furfuryl-3]-ester, 2,2-Dimethyl-3-(2',2'-difluorvinyl)-cyclopropancarbonsäure-[3-phenoxy-α-(2-methyl-propen-1-yl)-benzyl]-ester, 2,2-Dimethyl-3-(2',2'-difluorvinyl)-cyclopropancarbonsäure-(5-benzyl-α-isopropenyl-furfuryl-3)-ester, 2,2-Dimethyl-3-(2',2'-difluorvinyl)-cyclopropancarbonsäure-[5-benzyl-α-(n-buten-1-yl)-furfuryl-3]-ester, 2,2-Dimethyl-3-(2',2'-difluorvinyl)-cyclopropancarbonsäure-[3-phenoxy-α-(n-propen-1-yl)-benzyl]-ester, 2,2-Dimethyl-3-(2',2'-di-

fluorvinyl)-cyclopropancarbonsäure-[3-(4-chlorphenoxy)-α-(1-chlorpropen-1-yl)-benzyl]-ester, 2,2-Dimethyl-3-(2',2'-difluorvinyl)-cyclopropancarbonsäure-[3-phenoxy-α-(n-buten-1-yl)-benzyl]-ester, 2,2-Dimethyl-3-(2',2'-difluorvinyl)-cyclopropancarbonsäure-[5-benzyl-α-(2,2-dichlorvinyl)-furfuryl-3]-ester, 2,2-Dimethyl-3-(2',2'-dibromvinyl)-cyclopropancarbonsäure-(3-phenoxy-α-isopropenyl-benzyl)-ester, 2,2-Dimethyl-3-(2',2'-dibromvinyl)-cyclopropancarbonsäure-[3-phenoxy-α-(2,2-dichlorvinyl)-benzyl]-ester, 2,2-Dimethyl-3-(2',2'-dibromvinyl)-cyclopropancarbonsäure-[5-benzyl-α-(n-propen-1-yl)-furfuryl-3]-ester, 2,2-Dimethyl-3-(2',2'-dibromvinyl)-cyclopropancarbonsäure-[5-(4-fluorphenyl)-α-vinyl-furfuryl-3]-ester, 2,2-Dimethyl-3-(2',2'-dibromvinyl)-cyclopropancarbonsäure-(3-benzyl-α-vinyl-benzyl)-ester, 2-(4-Chlorphenyl)-isovaleriansäure-(3-phenylthio-α-vinyl-benzyl)-ester, 2-(4-Chlorphenyl)-isovaleriansäure-[3-phenoxy-α-(1-chlorvinyl)-4-fluor-benzyl]-ester, 2-(4-Chlorphenyl)-isovaleriansäure-[3-(4-fluorphenoxy)-α-vinyl-benzyl]-ester, 2-(4-Chlorphenyl)-isovaleriansäure-(3-phenoxy-α-trifluorvinyl-benzyl)-ester, 2,2-Dimethyl-3-(2',2'-dichlorvinyl)-cyclopropancarbonsäure-(3-phenoxy-α-isopropenyl-benzyl)-ester, 2,2-Dimethyl-3-(2',2'-dichlorvinyl)-cyclopropancarbonsäure-[3-phenoxy-α-(2,2-dichlorvinyl)-benzyl]-ester, 2,2-Dimethyl-3-(2',2'-dichlorvinyl)-cyclopropancarbonsäure-[5-benzyl-α-(n-propen-1-yl)-furfuryl-3]-ester, 2,2-Dimethyl-3-(2',2'-dichlorvinyl)-cyclopropancarbonsäure-[5-(4-fluorphenyl)-α-vinyl-furfuryl-3]-ester, 2,2-Dimethyl-3-(2',2'-dichlorvinyl)-cyclopropancarbonsäure-(3-benzyl-α-vinyl-benzyl)-ester, 2,2-Dimethyl-3-(2',2'-dichlorvinyl)-cyclopropancarbonsäure-(3-phenylthio-α-vinyl-benzyl)-ester, 2,2-Dimethyl-3-(2',2'-dichlorvinyl)-cyclopropancarbonsäure-[3-phenoxy-α-(1-chlorvinyl)-4-fluor-benzyl]-ester, 2,2-Dimethyl-3-(2',2'-dichlorvinyl)-cyclopropancarbonsäure-[3-(4-fluorphenoxy)-α-vinyl-

-benzyl]-ester, 2,2-Dimethyl-3-(2',2'-dichlorvinyl)-cyclopropancarbonsäure-(3-phenoxy-α-trifluorvinyl-benzyl)-ester, 2,2-Dimethyl-3-(2',2'-dichlorvinyl)-cyclopropancarbonsäure-[3-(4-chlorphenoxy)-α-(1-chlor-n-propen-1-yl)-benzyl]-ester, 2-(4-Chlorphenyl)-isovaleriansäure-[3-phenoxy-α-(n-propen-1-yl)-benzyl]-ester, 2-(4-Chlorphenyl)-isovaleriansäure-[3-(4-chlorphenoxy)-α-(1-chlorpropen-1-yl)-benzyl]-ester, 2-(4-Chlorphenyl)-isovaleriansäure-[3-phenoxy-α-(n-buten-1-yl)-benzyl]-ester, 2-(4-Chlorphenyl)-isovaleriansäure-[5-benzyl-α-(2,2-dichlorvinyl)-furfuryl-3]-ester, 2,2-Dimethyl-(2',2'-dibromvinyl)-cyclopropancarbonsäure-(5-benzyl-α-vinyl-furfuryl-3)-ester, 2,2-Dimethyl-(2',2'-dibromvinyl)-cyclopropancarbonsäure-[5-benzyl-α-(2-methyl-propen-1-yl)-furfuryl-3]-ester, 2,2-Dimethyl-(2',2'-dibromvinyl)-cyclopropancarbonsäure-[3-phenoxy-α-(2-methyl-propen-1-yl)-benzyl]-ester, 2,2-Dimethyl-(2',2'-dibromvinyl)-cyclopropancarbonsäure-(5-benzyl-α-isopropenyl-furfuryl-3)-ester, 2,2-Dimethyl-(2',2'-dibromvinyl)-cyclopropancarbonsäure-[5-benzyl-α-(n-buten-1-yl)-furfuryl-3]-ester, 2,2-Dimethyl-(2',2'-dibromvinyl)-cyclopropancarbonsäure-[3-phenoxy-α-(n-propen-1-yl)-benzyl]-ester, 2,2-Dimethyl-(2',2'-dibromvinyl)-cyclopropancarbonsäure-[3-phenoxy-α-(n-buten-1-yl)-benzyl]-ester, 2,2-Dimethyl-(2',2'-dibromvinyl)-cyclopropancarbonsäure-[5-benzyl-α-(2,2-dichlorvinyl)-furfuryl-3]-ester, 2,2-Dimethyl-3-(2',2'-difluorvinyl)-cyclopropancarbonsäure-(3-phenoxy-α-isopropenyl-benzyl)-ester, 2,2-Dimethyl-3-(2',2'-difluorvinyl)-cyclopropancarbon-[3-phenoxy-α-(2,2-dichlorvinyl)-benzyl]-ester, 2,2-Dimethyl-3-(2',2'-difluorvinyl)-cyclopropancarbonsäure-[5-benzyl-α-(n-propen-1-yl)-furfuryl-3]-ester, 2,2-Dimethyl-3-(2',2'-difluorvinyl)-cyclopropancarbonsäure-[5-(4-fluorphenyl)-α-vinyl-furfuryl-3]-ester, 2,2-Dimethyl-3-(2',2'-difluorvinyl)-cyclopropancarbonsäure-(3-benzyl-α-vinyl-benzyl)-ester, 2,2-Dimethyl-3-(2',2'-difluor-

vinyl)-cyclopropancarbonsäure-(3-phenylthio-α-vinyl-benzyl)-ester, 2,2-Dimethyl-3-(2',2'-difluorvinyl)-cyclopropancarbonsäure-[3-phenoxy-α-(1-chlorvinyl)-4-fluor-benzyl]-ester, 2,2-Dimethyl-3-(2',2'-difluorvinyl)-cyclopropancarbonsäure-[3-(4-fluorphenoxy)-α-vinyl-benzyl]-ester, 2,2-Dimethyl-3-(2',2'-difluorvinyl)-cyclopropancarbonsäure-(3-phenoxy-α-trifluorvinyl-benzyl)-ester, 2,2-Dimethyl-3-(2',2'-dibromvinyl)-cyclopropancarbonsäure-(3-phenylthio-α-vinyl-benzyl)-ester, 2,2-Dimethyl-3-(2',2'-dibromvinyl)-cyclopropancarbonsäure-[3-phenoxy-α-(1-chlorvinyl)-4-fluor-benzyl]-ester, 2,2-Dimethyl-3-(2',2'-dibromvinyl)-cyclopropancarbonsäure-[3-(4-fluorphenoxy)-α-vinyl-benzyl]-ester, 2,2-Dimethyl-3-(2',2'-dibromvinyl)-cyclopropancarbonsäure-(3-phenoxy-α-trifluorvinyl-benzyl)-ester, 2,2-Dimethyl-3-(2',2'-dibromvinyl)-cyclopropancarbonsäure-[3-(4-chlorphenoxy)-α-(1-chlor-n-propen-1-yl)-benzyl]-ester, 2-(4-Chlorphenyl)-isovaleriansäure-(5-benzyl-α-vinyl-furfuryl-3)-ester, 2-(4-Chlorphenyl)-isovaleriansäure-[5-benzyl-α-(2-methyl-propen-1-yl)-furfuryl-3]-ester, 2-(4-Chlorphenyl)-isovaleriansäure-[3-phenoxy-α-(2-methyl-propen-1-yl)-benzyl]-ester, 2-(4-Chlorphenyl)-isovaleriansäure-(5-benzyl-α-isopropenyl-furfuryl-3)-ester, 2-(4-Chlorphenyl)-isovaleriansäure-[5-benzyl-α-(n-buten-1-yl)-furfuryl-3]-ester, 2,2-Dimethyl-3-(2',2'-dichlorvinyl)-cyclopropancarbonsäure-(5-benzyl-α-vinyl-furfuryl-3)-ester, 2,2-Dimethyl-3-(2',2'-dichlorvinyl)-cyclopropancarbonsäure-[5-benzyl-α-(2-methyl-propen-1-yl)-furfuryl-3]-ester, 2,2-Dimethyl-3-(2',2'-dichlorvinyl)-cyclopropancarbonsäure-[3-phenoxy-α-(2-methyl-propen-1-yl)-benzyl]-ester, 2,2-Dimethyl-3-(2',2'-dichlorvinyl)-cyclopropancarbonsäure-(5-benzyl-α-isopropenyl-furfuryl-3)-ester, 2,2-Dimethyl-3-(2',2'-dichlorvinyl)-cyclopropancarbonsäure-[5-benzyl-α-(n-buten-1-yl)-furfuryl-3]-ester, 2,2-Dimethyl-3-(2',2'-dichlorvinyl)-cyclopropancarbonsäure-[3-phenoxy-α-(n-

-propen-1-yl)-benzyl]-ester, 2,2-Dimethyl-3-(2',2'-dichlorvinyl)-cyclopropancarbonsäure-[3-phenoxy-α-(n-buten-1-yl)-benzyl]-ester, 2,2-Dimethyl-3-(2',2'-dichlorvinyl)-cyclopropancarbonsäure-[5-benzyl-α-(2,2-dichlorvinyl)-furfuryl-3]-ester, 2-(4-Chlorphenyl)-isovaleriansäure-(3-phenoxy-α-isopropenyl-benzyl)-ester, 2-(4-Chlorphenyl)-isovaleriansäure-[3-phenoxy-α-(2,2-dichlorvinyl)-benzyl]-ester, 2-(4-Chlorphenyl)-isovaleriansäure-[5-benzyl-α-(n-propen-1-yl)-furfuryl-3]-ester, 2-(4-Chlorphenyl)-isovaleriansäure-[5-(4-fluorphenyl)-α-vinyl-furfuryl-3]-ester, 2-(4-Chlorphenyl)-isovaleriansäure-(3-benzyl-α-vinyl-benzyl)-ester. Es versteht sich von selbst, daß die Bezeichnung der Ester sämtliche mögliche Isomere umfaßt.

Weiterhin wurde gefunden, daß die neuen Ester der Formel I durch Umsetzung von Säurehalogeniden der Formel II

Y-CO-Hal II,

in der
Y die oben genannten Bedeutungen hat und Hal für Halogen, insbesondere für Chlor, steht, in Gegenwart eines säurebindenden Mittels mit Verbindungen der Formel III

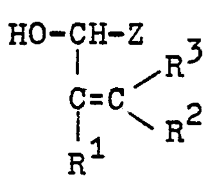
III,

in der
$R^1$, $R^2$, $R^3$ und Z die oben genannten Bedeutungen haben, erhalten werden.

Darüber hinaus ist es möglich, die neuen Ester der Formel I durch Umsetzung von Säuren der Formel IV

Y-COOH IV,

in der Y die oben genannten Bedeutungen hat, mit Halogeniden der Formel V

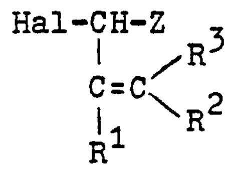

V,

in der $R^1$, $R^2$, $R^3$ und Z die oben genannten Bedeutungen haben und Hal für Halogen, insbesondere Chlor, steht, in Gegenwart eines säurebindenden Mittels herzustellen.

Weitere Synthesewege sind die Umsetzung von Säuren der Formel IV mit Verbindungen der Formel III in Gegenwart eines wasserbindenden Mittels und die Umsetzung von Alkylestern der Formel VI

Y-COOR VI

in der Y die oben genannten Bedeutungen hat und R für unverzweigtes oder verzweigtes Alkyl mit 1 bis 5 Kohlenstoffatomen steht, mit Verbindungen der Formel III in Gegenwart eines für Umesterungen üblichen Katalysators.

Die einzelnen Reaktionswege lassen sich durch folgende Reaktionsgleichungen darstellen:

a) 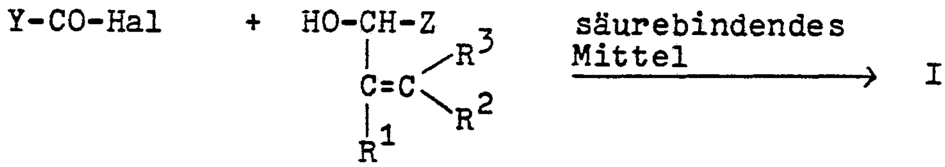


II III

b) 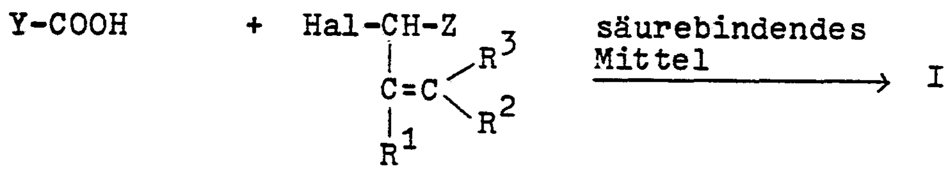


IV V

c) 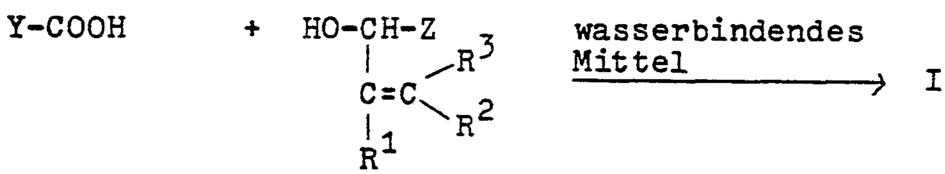


IV III

d) 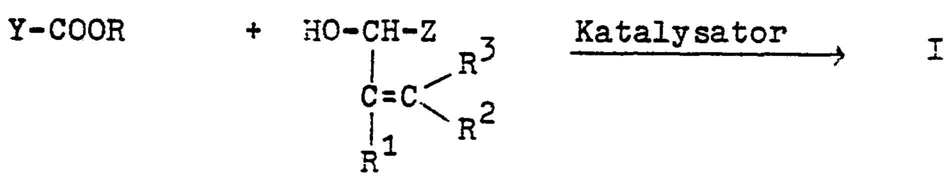


VI III

Als säurebindende Mittel für die Synthesewege a) und b) kommen organische Basen, beispielsweise tertiäre Amine, wie Triäthylamin, Trimethylamin, Dimethylanilin, Dimethylbenzylamin, oder Pyridin, sowie anorganische Basen, wie Hydroxide, Oxide, Hydrogencarbonate oder Carbonate von Alkali- oder Erdalkalimetallen, beispielsweise Natriumhydroxid, Kaliumhydroxid, Calciumhydroxid, Calciumhydrogencarbonat, Kaliumcarbonat, und Alkoholate oder Hydride der Alkalimetalle, beispielsweise Natriumhydrid oder Kalium-t-butylat, in Betracht. Das säurebindende Mittel wird in mindestens molaren Mengen, bezogen auf die Verbindung der Formel II bzw. V, eingesetzt.

Für die Umsetzung entsprechend Reaktionsgleichung c) eignen sich als wasserbindende Mittel beispielsweise Dicyclohexylcarbodiimid, anorganische Mineralsäuren, wie Schwefelsäure, Phosphorsäure, saure Ionenaustauscher oder p-Toluolsulfonsäure. Das wasserbindende Mittel wird dabei in Mengen von mindestens 0,02, zweckmäßigerweise 0,05 bis 0,4 Mol, bezogen auf die im Unterschuß vorhandene Reaktionskomponente III bzw. IV, verwendet.

Geeignete Katalysatoren für die Umesterung entsprechend Reaktionsgleichung d) sind Hydride oder Alkoholate der Alkalimetalle, wie Natriumhydrid, Natriumäthylat, und Triphenyl-natrium. Ebenso geeignet sind Alkoholate der Elemente der Gruppe IV b des Periodensystems, wie Titantetramethylat, Titantetraäthylat. Pro Mol Ester der Formel VI werden zweckmäßigerweise 0,02 bis 0,4 Mol Katalysator zugegeben.

Alle Herstellungsverfahren a) bis d) werden bei Reaktionstemperaturen im Bereich zwischen -10 und +150°C, drucklos oder unter Druck, durchgeführt.

Zur Durchführung der Verfahren setzt man die Reaktionskomponenten der Formeln II und III, IV und V, IV und III sowie VI und III vorzugsweise in äquimolaren Verhältnissen ein. Ein Überschuß der einen oder anderen Komponente bringt keine wesentlichen Vorteile. Die Umsetzung verläuft praktisch quantitativ.

Die Verfahren a) bis d) werden zweckmäßigerweise in Gegenwart geeigneter Lösungs- oder Verdünnungsmittel durchgeführt. Als solche kommen praktisch alle inerten organischen Solventien in Betracht. Hierzu gehören acyclische und cyclische Äther, wie Diäthyläther, Tetrahydrofuran, Dioxan, alkylierte aliphatische und alicyclische Carbon-

säureamide, wie Dimethylformamid, N-Methylpyrrolidon, aliphatische und aromatische, gegebenenfalls chlorierte Kohlenwasserstoffe, wie Toluol, Xylole, Chloroform, n-Hexan, Cyclohexan, Chlorbenzol, Ketone, wie Aceton, Methyläthylketon, Nitrile, wie Acetonitril, ferner Dimethylsulfoxid, Hexamethylphosphorsäuretriamid. Auch Gemische dieser Lösungsmittel können verwendet werden.

Das Verfahren entsprechend Reaktionsgleichung a) kann auch in wäßriger Lösung durchgeführt werden. Als Zweiphasenreaktion können die Verfahren a) und b) durchgeführt werden, wobei als wasserunlösliche organische Phase beispielsweise Äther, aliphatische und aromatische, gegebenenfalls chlorierte Kohlenwasserstoffe, insbesondere Toluol oder Chloroform, und Ketone, wie Methyläthylketon, verwendet werden können.

Werden bei der Herstellung der Ester der Formel I nicht einheitliche, optisch aktive bzw. cis- oder transisomere Ausgangsmaterialien verwendet, liegen die Endprodukte als Gemische verschiedener optisch aktiver Isomeren sowie von cis/trans-Isomeren vor. Diese Isomerengemische können nach bekannten Methoden in die einheitlichen Isomeren aufgetrennt werden. Unter dem Begriff Ester der Formel I versteht man sowohl die reinen Isomeren als auch deren Gemische.

Die Ausgangsverbindungen der Formel II sind bekannt (GB-PS 1 446 304, US-PS 3 981 903). Die Verbindungen der Formel III können nach an sich bekannten Verfahren hergestellt werden, beispielsweise durch Umsetzung von Aldehyden mit Metallorganylen oder durch Anlagerung von Alkinen an Aldehyde mit anschließender partieller Hydrierung (Houben-Weyl, Methoden der organischen Chemie, Bd. V/1b, S. 775-790, Georg Thieme-Verlag, Stuttgart, 1972). Verbindungen der Formeln IV, V und-VI sind ebenfalls bekannt und

können nach an sich bekannten Verfahren hergestellt werden (US-PS 3 979 519, US-PS 3 981 903, BE-PS 801 946, DE-OS 23 65 555, DE-OS 22 31 312).

Die folgenden Beispiele erläutern die Herstellung der erfindungsgemäßen Ester der Formel I.

Beispiel 1

a) 3-Phenoxy-α-vinyl-benzylalkohol

30 ml einer 1,6 molaren Lösung von Vinylmagnesiumchlorid in Tetrahydrofuran werden in 100 ml absolutem Tetrahydrofuran bei 0°C vorgelegt. Unter Rühren und Feuchtigkeitsausschluß tropft man 9,8 g (0,05 Mol) 3-Phenoxybenzaldehyd in 100 ml absolutem Tetrahydrofuran zu. Man läßt 3 Stunden bei 25°C rühren und zersetzt das Reaktionsgemisch vorsichtig mit einer kalt gesättigten Ammoniumchloridlösung und einigen Millilitern 10 %iger Salzsäure, bis sich der Niederschlag auflöst. Dann wird dreimal mit Äther ausgeschüttelt, die vereinigten Ätherextrakte werden mit Natriumhydrogensulfitlösung, Natriumhydrogencarbonatlösung und Wasser gewaschen, über Natriumsulfat getrocknet und unter vermindertem Druck eingeengt.

Man erhält 9,4 g eines gelblichen Öls, das für die weitere Umsetzung rein genug ist.

| | | | |
|---|---|---|---|
| Ber.: | C 79,62 | H 6,24 | O 14,14 |
| Gef.: | C 79,3 | H 6,1 | O 14,5 |

b) 2,2-Dimethyl-3-(2',2'-dibromvinyl)-cyclopropan-carbonsäure-(3-phenoxy-α-vinyl-benzyl)-ester

Zu 5,1 g (0,05 Mol) Pyridin, 100 ml abolutem Äther und 11,4 g (0,05 Mol) 3-Phenoxy-α-vinyl-benzylalkohol werden bei 0 bis 5°C 15,8 g (0,05 Mol) 2,2-Dimethyl-3-(2',2'-dibromvinyl)-cyclopropancarbonsäurechlorid unter Rühren zugetropft.

Nach 12-stündigem Rühren bei Raumtemperatur wird das Reaktionsgemisch abgesaugt und das Filtrat nacheinander mit 2n Natriumhydrogencarbonatlösung, 2n Salzsäure und Wasser gewaschen. Nach dem Trocknen über Natriumsulfat wird unter vermindertem Druck eingeengt und das erhaltene Öl säulenchromatographisch über Kieselgel mit Toluol als Elutionsmittel gereinigt.

Man erhält ein Isomerengemisch der Formel

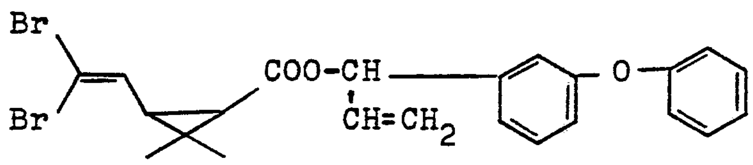

als gelbliches Öl mit einer Refraktion von $n_D^{28}$ = 1,5823; Ausbeute: 8 g.

Beispiel 2

a) 3-Phenoxy-(α-trifluorvinyl)-benzylalkohol

In 100 ml absolutem Äther werden bei -78°C 19,32 g (0,12 Mol) 1-Brom-1,2,2-trifluorethen eingegast. Unter Stickstoffspülung tropft man bei dieser Temperatur 66,6 ml einer 1,5n Butlyllithiumlösung in n-Hexan zu. Zu diesem Reaktionsgemisch wird dann nach 30 Minuten

eine Lösung von 19,8 g (0,1 Mol) m-Phenoxybenzaldehyd in 100 ml absolutem Äther getropft. Nachdem das Reaktionsgemisch sich auf Raumtemperatur erwärmt hat, wird es mit einer gesättigten Ammoniumchloridlösung und verdünnter Salzsäure hydrolysiert. Die Ätherphase wird abgetrennt, mit einer zu Natriumhydrogencarbonat-Lösung und Wasser gewaschen, getrocknet und eingeengt. Man erhält 26 g eines dunkelroten Öls, das für die weiteren Umsetzungen rein genug ist.

Eine kleine Menge wird über eine Kieselgelsäule mit Cyclohexan/Aceton (85/15) gereinigt. Man isoliert ein rötliches Öl mit einer Refraktion von $n_D^{23} = 1{,}5442$.

b) 2,2-Dimethyl-3-(2',2'-dichlorvinyl)-cyclopropan-carbonsäure-[3-phenoxy-(α-trifluorvinyl)-benzyl]-ester

14 g (0,05 Mol) 3-Phenoxy-(α-trifluorvinyl)-benzylalkohol in 150 ml absolutem Äther und 5,6 g (0,06 Mol) γ-Picolin werden bei 0 bis 5°C vorgelegt. Dazu tropft man langsam eine Lösung von 11,5 g (0,05 Mol) 2,2-Dimethyl-3-(2',2'-dichlorvinyl)-cyclopropancarbonsäurechlorid in 20 ml absolutem Äther. Man läßt über Nacht bei Raumtemperatur rühren, filtriert und wäscht die Ätherlösung mit 2n Salzsäure, 2n Natriumhydrogencarbonatlösung und Wasser, trocknet über Natriumsulfat und engt unter vermindertem Druck ein. Das erhaltene Öl wird über eine Kieselgelsäule mit Cyclohexan/Aceton (85/15) als Elutionsmittel gereinigt; Ausbeute 10,2 g; $n_D^{25} = 1{,}5390$.

Beispiel 3

a) 2-Benzyl-(α-vinyl)-benzylalkohol

30 ml einer 1,6 molaren Lösung von Vinylmagnesiumchlorid in Tetrahydrofuran werden in 100 ml absolutem Tetrahydrofuran bei 0°C vorgelegt. Unter Rühren und Feuchtigkeitsausschluß tropft man 9,8 g (0,05 Mol) 2-Benzylbenzaldehyd in 100 ml absolutem Tetrahydrofuran zu. Man läßt 3 Stunden bei 25°C rühren und zersetzt das Reaktionsgemisch vorsichtig mit einer kalt gesättigten Ammoniumchloridlösung und einigen Millilitern 10 %iger Salzsäure, bis sich der Niederschlag auflöst. Dann wird dreimal mit Äther ausgeschüttelt, die vereinigten Ätherphasen mit Natriumhydrogensulfitlösung, Natriumhydrogencarbonatlösung und Wasser gewaschen, über Natriumsulfat getrocknet und unter vermindertem Druck eingeengt. Man erhält 9,4 g eines gelblichen Öls, das für die weiteren Umsetzungen rein genug ist; $n_D^{21}$ = 1,5855.

b) 2,2-Dimethyl-3-(2',2'-dichlorvinyl)-cyclopropancarbonsäure-[2-benzyl-(α-vinyl)-benzyl]-ester

Zu 4,6 g (0,05 Mol) γ-Picolin, 100 ml absolutem Äther und 8,5 g (0,38 Mol) 2-Benzyl-(α-vinyl)-benzylalkohol werden bei 0 bis 5°C 91 g (0,04 Mol) 2,2-Dimethyl-3-(2',2'-dichlorvinyl)-cyclopropancarbonsäurechlorid unter Rühren zugetropft. Nach 12-stündigem Rühren bei 25°C wird das Reaktionsgemisch abgesaugt und das Filtrat nacheinander mit einer 2n-Natriumhydrogencarbonatlösung, 2n-Salzsäure und Wasser gewaschen. Nach dem Trocknen über Natriumsulfat wird unter vermindertem Druck eingeengt und das erhaltene Öl säulenchromatographisch über Kieselgel mit Toluol als Elutionsmittel gereinigt.

Man erhält 10,3 g eines Isomerengemisches der Formel

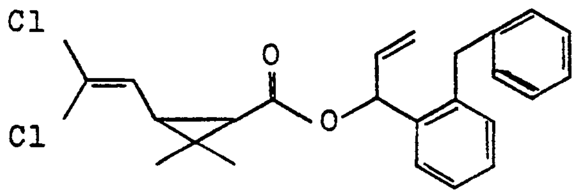

als gelbliches Öl; $n_D^{21}$ = 1,5678

Beispiel 4

a) 2-Benzyl-(α-trifluorvinyl)-benzylalkohol

In 100 ml absol. Äther werden bei -78°C 19,32 g (0,12 Mol) 1-Brom-1,2,2-trifluoräthen eingegast. Unter Stickstoffspülung tropft man gleichzeitig bei dieser Temperatur in mehreren Portionen 66,6 ml einer 1,5n Butyllithiumlösung in n-Hexan und 19,6 g (0,1 Mol) 2-Benzyl-benzaldehyd in 100 ml absolutem Äther zu. Man läßt langsam auf Raumtemperatur kommen und hydrolysiert mit einer gesättigten Ammoniumchloridlösung und verdünnter Salzsäure. Die Ätherphase wird abgetrennt, mit einer 2n Natriumhydrogencarbonatlösung und Wasser gewaschen, getrocknet und eingeengt.

Die erhaltenen 26,7 g eines dunkelroten Öls sind für die weiteren Umsetzungen rein genug.

Eine kleine Menge wird über eine Kieselgelsäule mit Cyclohexan/Aceton = 85/15 gereinigt. Man isoliert ein rötliches Öl mit einer Refraktion von $n_D^{23}$ = 1,5388.

b) 2,2-Dimethyl-3-(2',2'-dibromvinyl)-cyclopropan-carbonsäure-[2-benzyl-(α-trifluorvinyl)-benzyl]-ester

11,1 g (0,04 Mol) 2-Benzyl-(α-trifluorvinyl)-benzylalkohol in 100 ml absol. Äther und 4,6 g (0,05 Mol) γ-Picolin werden bei 0 bis 5°C vorgelegt. Dazu tropft man langsam eine Lösung von 19,0 g (0,05 Mol) 2,2-Dimethyl-3-(2',2'-dibromvinyl)-cyclopropancarbonsäurechlorid in 20 ml absolutem Äther. Man läßt über Nacht bei Raumtemperatur rühren, filtriert und wäscht die Ätherlösung mit 2n Salzsäure, 2n Natriumhydrogencarbonat und Wasser, trocknet über Natriumsulfat und engt unter vermindertem Druck ein. Das erhaltene Öl wird über eine Kieselgelsäule mit Cyclohexan/Aceton = 85/15 als Elutionsmittel gereinigt.

Man erhält 10,4 g eines Öls der Formel

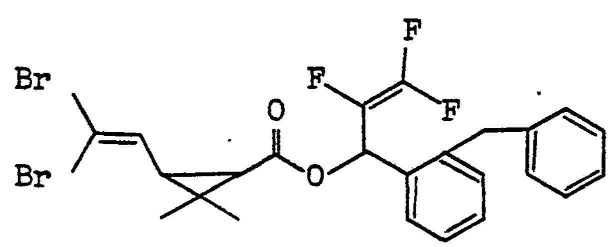

als Isomerengemisch mit einer Refraktion von $n_D^{21} = 1,5561$.

Analog können folgende Verbindungen hergestellt werden:

Y-COO-CH-Z
CH=CH2

| Nr. | Y | Z | $n_D$ |
|---|---|---|---|
| 5 | 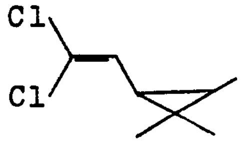 | 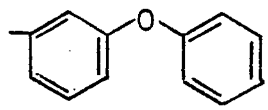 | $n_D^{23}$ = 1,5628 |
| 6 | 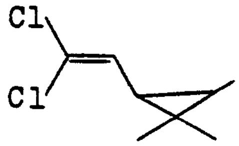 | 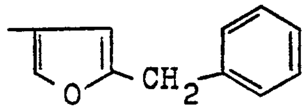 | $n_D^{23}$ = 1,5595 |
| 7 | 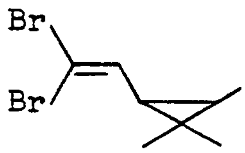 | 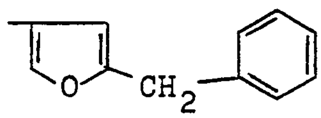 | $n_D^{23}$ = 1,5655 |
| 8 | 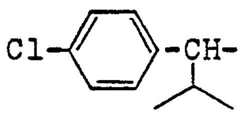 | 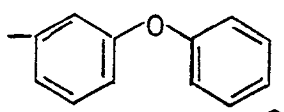 | $n_D^{25}$ = 1,5603 |
| 9 | 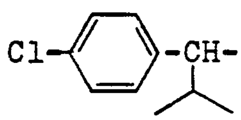 | 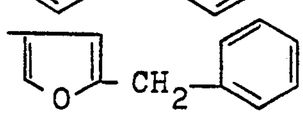 | $n_D^{23}$ = 1,5630 |
| 10 | 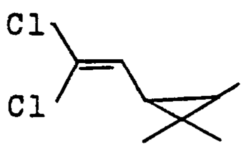 | 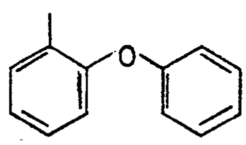 | $n_D^{23}$ = 1,5621 |
| 11 | 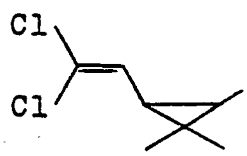 | 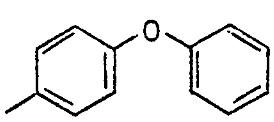 | $n_D^{21}$ = 1,5672 |
| 12 | 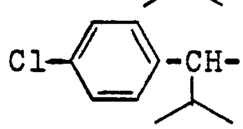 | 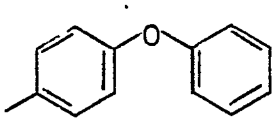 | $n_D^{21}$ = 1,5721 |
| 13 | 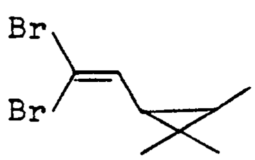 | 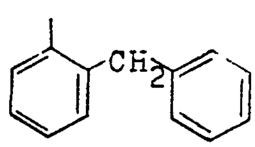 | $n_D^{21}$ = 1,5810 |

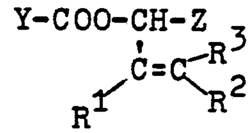

| Nr. | Y | Z | $R^1$ | $R^2$ | $R^3$ | $n_D$ |
|---|---|---|---|---|---|---|
| 14 | 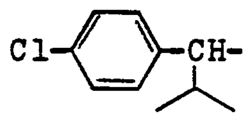 | 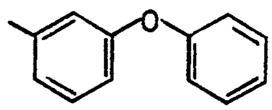 | F | F | F | $n_D^{25}$ = 1,5422 |
| 15 | 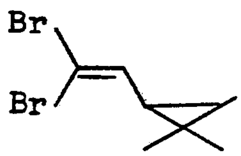 | 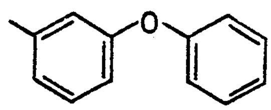 | F | F | F | $n_D^{22}$ = 1,5515 |
| 16 | 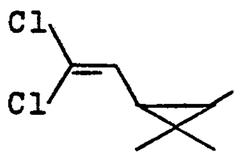 | 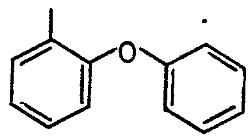 | F | F | F | $n_D^{23}$ = 1,5358 |
| 17 | 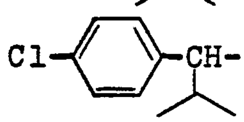 | 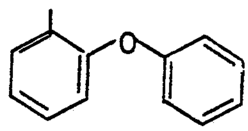 | F | F | F | $n_D^{23}$ = 1,5351 |
| 18 | 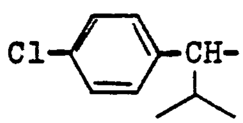 | 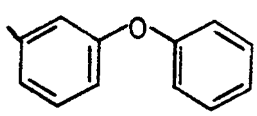 | H | F | F | $n_D^{22}$ = 1,5553 |
| 19 | 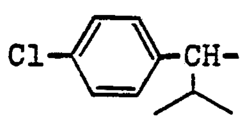 | 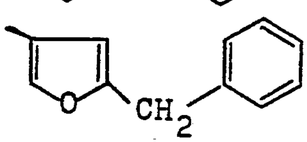 | F | F | F | $n_D^{22}$ = 1.5320 |
| 20 | 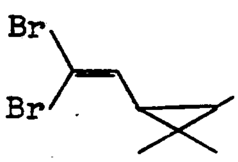 | 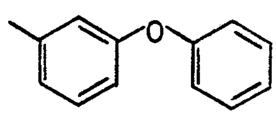 | H | F | F | $n_D^{22}$ = 1,5626 |
| 21 | 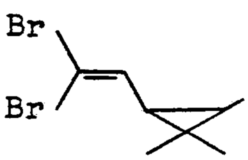 | 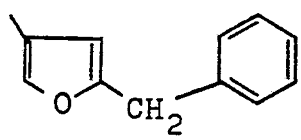 | F | F | F | $n_D^{22}$ = 1.5452 |
| 22 | 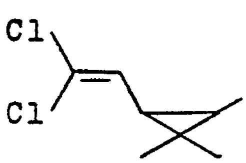 | 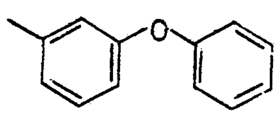 | H | F | F | $n_D^{23}$ = 1,5572 |

| Nr. | Y | Z | $R^1$ | $R^2$ | $R^3$ | $n_D$ |
|---|---|---|---|---|---|---|
| 23 | 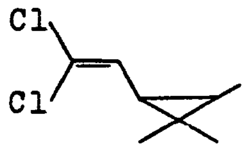 | 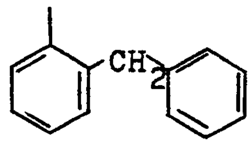 | F | F | F | $n_D^{22}$ = 1.5426 |
| 24 | 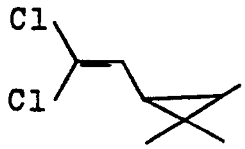 | 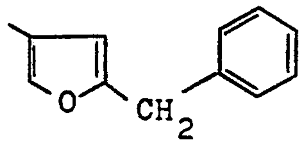 | F | F | F | $n_D^{22}$ = 1.5223 |

Die erfindungsgemäßen Carbonsäureester sind geeignet, Schädlinge aus der Klasse der Insekten und Spinnentiere wirksam zu bekämpfen.

Zu den schädlichen Insekten gehören aus der Ordnung der Schmetterlinge (Lepidoptera) beispielsweise Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria Mellonella (Wachsmotte), Malacosoma neustria (Ringelspinner), Dendrolimus pini (Kiefernspinner), Thaumatopoea pityocampa (Pinienprozessionsspinner), Phalera bucephala (Mondfleck), Cheimatobia brumata (Kleiner Frostspanner), Hibernia defoliaria (Großer

Frostspanner), Bupalus piniarus (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling), Aporia crataegi (Baumweißling);

aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agricotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varicestris (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagi (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-punctata (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Linierter Blattrandkäfer), Otiorrhynchus sulcatus (Gefurchter Lappenrüßler), Otiorrhynchus ovatus (Erdbeerwurzelrüßler), Hylobies abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus

assimilis (Kohlschotenrüßler), Ceuthorrhynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefruchter Wandgärtner);

aus der Ordnung der Zweiflügler (Diptera) beispielsweise Mayetiola destructor (Hessenfliege), Dasyneura brassicae (Kohlschoten-Gallmücke), Contarinia tritici (Gelbe Weizen--Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeer-Fruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hyoscyami (Rübenfliege), Musca domestica (Stubenfliege);

aus der Ordnung der Hautflügler (Hymenoptera) beispielsweise Athalia rosae (Rübenblattwespe), Hoplocampa minuta (Pflaumensägewespe), Monomorium pharaonis (Pharaoameise), Solenopsis geminata (Feuerameise), atta sexdens (Blattschneiderameise);

aus der Ordnung der Wanzen (Heteroptera) beispielsweise Nezara viridula (Grüne Reiswanze), Eurygaster integriceps (Asiatische Getreidewanze), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);

aus der Ordnung der Pflanzensauger (Homoptera) beispielsweise Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlaus), Aphidula nastrutii (Kreuzdornblattlaus), Cerosipha gossypii (Gurkenblattlaus), Sappaphis mali (Rosige Apfellaus), Sappaphis mala (Mehlige Birnblattlaus), Dysphis radicola (Mehlige Apfelfalterlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Dysaulacorthum pseudosolani (Gefleckte Kartoffellaus), Acyrthosiphon onobrychis (Grüne Erbsenlaus), Macrosiphon rasae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus);

aus der Ordnung der Termiten (Isoptera) beispielsweise Reticulitermes lucifugus, Termes natalensis, Calotermes flavicollis, Leucotermes flavipes;

aus der Ordnung der Geradflügler (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrekke), Melanoplus spretus (Felsengebirgsheuschrecke), Melano-

plus femur-rubrum (Rotbeinige Heuschrecke), Blatta orientalis (Küchenschabe), Blattella germanica (Deutsche Schabe), Periplaneta americana (Amerikanische Schabe), Blabera gigantea (Riesenschabe).

Zur Klasse der Spinnentiere (Arachnoidea) gehören Milben und Zecken (Acarina) beispielsweise Ixodes ricinus (Holzbock), Ornithodorus moubata, Amblyomma americanum, Dermacentor silvarum, Boophilus microplus, Tetranychus telarius, Tetranychus atlanticus, Tetranychus pacificus, Paratetranychus pilosus, Bryobia praetiosa.

Die erfindungsgemäßen Verbindungen können mit Erfolg im Pflanzenschutz sowie auf dem Hygiene-, Vorratsschutz- und Veterinärsektor als Schädlingsbekämpfungsmittel eingesetzt werden.

Die Wirkstoffe können als solche, in Form ihrer Formulierungen oder den daraus bereiteten Anwendungsmöglichkeiten, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Äthanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalin-

sulfonsäuren mit Phenol und Formaldehyd, Polyoxyäthylenoctylphenoläther, äthoxyliertes Isooctylphenol, Octylphenol, Nonylphenon, Alkylphenolpolyglykoläther, Tributylphenylpolyglykoläther, Alkylarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.% Wirkstoff, vorzugsweise zwischen 0,5 und 90 Gew.%.

Die Wirkstoffkonzentrationen in den anwendungsfertigen Zubereitungen können in größeren Bereichen variiert werden. Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit über 95 Gew.% Wirkstoff oder sogar den Wirkstoff ohne Zusätze auszubringen.

Beispiele für Formulierungen sind:

I. 3 Gewichtsteile 2,2-Dimethyl-3-(2',2'-dibromvinyl)-cyclopropancarbonsäure-(3-phenoxy-α-vinyl-benzyl)-ester werden mit 97 Gewichtsteilen feinteiligem Kaolin innig vermischt. Man erhält auf diese Weise ein Stäubemittel, das 3 Gewichtsprozent des Wirkstoffs enthält.

II. 30 Gewichtsteile 3'-Phenoxy-α'-vinyl-benzyl-α-isopropyl-4-chlorphenylacetat werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 20 Gewichtsteile 5'-Benzyl-α'-vinyl-furyl-3'-methyl-α-isopropyl-4-chlorphenylacetat werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile 2,2-Dimethyl-3-(2',2'-dichlorvinyl)-cyclopropancarbonsäure-[3-phenoxy-(α-trifluorvinyl)-benzyl]-ester werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Zu den Wirkstoffen können Öle verschiedenen Typs, Herbizide, Fungizide, andere Insektizide, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1 : 10 bis 10 : 1 zugemischt werden.

Beispielsweise können folgende Mittel zugemischt werden: 1,2-Dibrom-3-chlorpropan, 1,3-Dichlorpropen, 1,3-Dichlorpropen + 1,2-Dichlorpropan, 1,2-Dibrom-äthan, 2-sec.-Butyl-phenyl-N-methylcarbamat, o-Chlorphenyl-N-methylcarbamat, 3-Isopropyl-5-methylphenyl-N-methylcarbamat, o-Isopropoxyphenyl-N-methylcarbamat, 3,5-Dimethyl-4-methylmercapto-phenyl-N-methylcarbamat, 4-Dimethylamino-3,5-xylyl-N-methylcarbamat, 2-(1,3-Dioxolan-2-yl)-phenyl-N-methylcarbamat, 1-Naphthal-N-methylcarbamat, 2,3-Dihydro-2,2-dimethyl-benzofuran-7-yl-N-methylcarbamat, 2,2-Dimethyl-1,3-benzodioxol-4-yl-N-methylcarbamat, 2-Dimethylamino-5,6-dimethyl-4-pyrimidinyl-dimethylcarbamat, 2-Methyl-2-(methylthio)-propionaldehyd-O-(methylcarbamoyl)-oxim, S-Methyl-N-[(methylcarbamoyl)-oxy]-thio-acetimidat, Methyl-N',N'-dimethyl-N-[(methylcarbamoyl)oxy]-1-thiooxamidat, N-(2-Methyl-4-chlor-phenyl)-N',N'-dimethylformamidin, Tetrachlorthiophen, 1-(2,6-Difluor-benzoyl)-3-(4-chlor-phenyl)-harnstoff

O,O-Dimethyl-O-(p-nitrophenyl)-phosphorthioat, O,O-Diäthyl--O-(p-nitrophenyl)-phosphorthioat, O-Äthyl-O-(p-nitrophenyl)-phenyl-phosphonothioat, O,O-Dimethyl-O-(3-methyl-4--nitrophenyl)-phosphorthioat, O,O-Diäthyl-O-(2,4-dichlorphenyl)-phosphorthioat, O-Äthyl-O-(2,4-dichlorphenyl)-phenyl-phosphonothioat, O,O-Dimethyl-O-(2,4,5-trichlorphenyl)--phosphorthioat, O-Äthyl-O-(2,4,5-trichlorphenyl)-äthyl--phosphonothioat, O,O-Dimethyl-O-(4-brom-2,5-dichlorphenyl)--phosphorthioat, O,O-Dimethyl-O-(2,5-dichlor-4-jodphenyl)--phosphorthioat, O,O-Dimethyl-O-(3-methyl-4-methylthiophenyl)-phosphorthioat, O-Äthyl-O-(3-methyl-4-methylthiophenyl)-isopropyl-phosphoramidat, O,O-Diäthyl-O-[p-(methylsulfinyl)phenyl]-phosphorthioat, O-Äthyl-S-phenyl-äthyl-phosphonodithioat, O,O-Diäthyl-[2-chlor-1-(2,4-dichlorphenyl)--vinyl]-phosphat, O,O-Dimethyl-[-2-chlor-1-(2,4,5-trichlorphenyl)]-vinyl-phosphat, O,O-Dimethyl-S-(1'-phenyl)-äthylacetat-phosphordithioat, Bis-(dimethylamino)-fluorphosphinoxid, Octamethyl-pyrophosphoramid, O,O,O,O-Tetraäthyldithio-pyrophosphat, S-Chlormethyl-O,O-diäthyl-phosphordithioat, O-Äthyl-S,S-dipropyl-phosphordithioat, O,O-Dimethyl-O-2,2-dichlorvinyl-phosphat, O,O-Dimethyl-1,2-dibrom-2,2-dichloräthylphosphat, O,O-Dimethyl-2,2,2-trichlor--1-hydroxy-äthylphosphonat, O,O-Dimethyl-S-[1,2-biscarbäthoxy-äthyl-(1)]-phosphordithioat, O,O-Dimethyl-O-(1-methyl-2-carbmethoxy-vinyl)-phosphat, O,O-Dimethyl-S-(N-methyl-carbamoyl-methyl)-phosphordithioat, O,O-Dimethyl-S--(N-methyl-carbamoyl-methyl)-phosphorthioat, O,O-Dimethyl--S-(N-methoxyäthyl-carbamoyl-methyl)-phosphordithioat, O,O--Dimethyl-S-(N-formyl-N-methyl-carbamoylmethyl-phosphordithioat, O,O-Dimethyl-O-[1-methyl-2-(methyl-carbamoyl)-vinyl]-phosphat, O,O-Dimethyl-O-[(1-methyl-2-dimethylcarbamoyl)-vinyl]-phosphat, O,O-Dimethyl-O-[(1-methyl-2-chlor--2-diäthylcarbamoyl)-vinyl]-phosphat, O,O-Diäthyl-S-(äthylthio-methyl)-phosphordithioat, O,O-Diäthyl-S-[(p-chlorphenylthio)-methyl]-phosphordithioat, O,O-Dimethyl-S-(2-

-äthylthioäthyl)-phosphorthioat, O,O-Dimethyl-S-(2-äthylthioäthyl)-phosphordithioat, O,O-Dimethyl-S-(2-äthylsulfinyl-äthyl)-phosphorthioat, O,O-Diäthyl-S-(2-äthylthio-äthyl)-phosphordithioat, O,O-Diäthyl-S-(2-äthylsulfinyl-äthyl)-phosphorthioat, O,O-Diäthyl-thiophosphoryliminophenyl-acetonitril, O,O-Diäthyl-S-(2-chlor-1-phthalimidoäthyl)-phosphordithioat, O,O-Diäthyl-S-[6-chlor-benzoxazolon-(2)-yl(3)]-methyldithiophosphat, O,O-Dimethyl-S-[2-methoxy-1,3,4-thiadiazol-5-[4H]-onyl-(4)-methyl]-phosphordithioat, O,O-Diäthyl-O-[3,5,6-trichlor-pyridyl-(2)]-phosphorthioat, O,O-Diäthyl-O-(2-pyrazinyl)-phosphorthioat, O,O-Diäthyl-O-[2-isopropyl-4-methyl-pyrimidinyl(6)]-phosphorthioat, O,O-Diäthyl-O-[2-(diäthylamino)-6-methyl-4-pyrimidinyl]-thionophosphat, O,O-Dimethyl-S-(4-oxo-1,2,3-benzotriazin-3-[4H]-yl-methyl)-phosphordithioat, O,O-Dimethyl-S-[(4,6-diamino-1,3,5-triazin-2-yl)-methyl]-phosphordithioat, O,O-Diäthyl-(1-phenyl-1,2,4-triazol-3-yl)-thionophosphat, O,S-Dimethyl-phosphor-amido-thioat, O,S-Dimethyl-N-acetyl-phosphoramidothioat, γ-Hexachlorcyclohexan, 1,1-Di-(p-methoxyphenyl)-2,2,2-trichlor-äthan, 6,7,8,9,10,10-Hexachloro-1,5,5a,6,9,9a-hexahydro-6,9-methano-2,4,3-benzodioxa-thiepin-3-oxid, Pyrethrine, DL-2-Allyl-3-methyl-cyclopenten-(2)-on-(1)-yl-(4)-DL-cis,trans-chrysanthemat, 5-Benzyl-furyl-(3)-methyl-DL-cis,trans-chrysanthemat, 3-Phenoxybenzyl(±)-cis,trans-2,2-dimethyl-3-(2,2-dichlorvinyl)-cyclopropancarboxylat, α-Cyano-3-phenoxybenzyl(±)-cis,trans-2,2-dimethyl-3-(2,2-dichlorvinyl)-cyclopropancarboxylat, (s)-α-Cyano-3-phenoxybenzyl-cis(1R,3R)-2,2-dimethyl-3-(2,2-dibromvinyl)-cyclopropancarboxylat, 3,4,5,6-Tetrahydrophthalimidoäthyl-DL-cis,trans-chrysanthemat, 2-Methyl-5-(2-propinyl)-3-furylmethyl-chrysanthemat, α-Cyano-3-phenoxybenzyl-α-isopropyl-4-chlorphenylacetat.

Mit besonderem Vorteil werden Verbindungen der Formel I auch mit Substanzen kombiniert, welche einen synergisti-

schen oder verstärkenden Effekt auf Pyrethroide ausüben. Beispiele solcher Verbindungen sind u.a. Piperonylbutoxid, Propinyläther, Propinyloxime, Propinylcarbamate und Propinylphosphonate, 2-(3,4-Methylendioxyphenoxy)-3,6,9-trioxaundecan, S,S,S-Tributylphosphorotrithioate, 1,2-Methylendioxy-4-(2-(octylsulfonyl)-propyl)-benzol, 1-n-Dodecylimidazol, 1-(1,5,9-Trimethyldecyl)-imidazol, 1-[2-Chlor-2-(4-fluorphenyl)-äthyl]-1,2,4-triazol, 1-(2-Phenyläthyl)-1,2,4-triazol, 1-(2-Chlor-2-phenyl-äthyl)-1,2,4-triazol, 1-(3-Phenyl-n-propyl)-1,2,4-triazol.

Die folgenden Beispiele belegen die biologische Wirkung der neuen Ester. Vergleichsmittel ist 2,2-Dimethyl-3-(2',2'-dimethylvinyl)-cyclopropancarbonsäure-(3-phenoxy-benzyl)-ester (Agr. Biol. Chem 37, 2681 (1973)). Die Numerierung der Wirkstoffe entspricht den Herstellungsbeispielen.

Beispiel A

Kontaktwirkung auf Stubenfliegen (Musca domestica); Applikationstest

4 Tage alte Imagines erhalten in leichter $CO_2$-Narkose 1 µl der acetonischen Lösung des Wirkstoffs auf das ventrale Abdomen appliziert. Hierzu wird eine Mikrometerspritze verwendet. Je 20 Versuchstiere mit gleicher Behandlung bringt man dann in einen Folienbeutel (Volumen ca. 500 ml).

Nach 4 Stunden zählt man die Tiere in Rückenlage aus und ermittelt graphisch die LD 50.

| Wirkstoff Nr. | LD 50 |
|---|---|
| 1 | 0,014 /ug/Fliege |
| 5 | 0,0098 /ug/Fliege |
| Vergleichsmittel | 0,017 /ug/Fliege |

Beispiel B

Kontaktwirkung auf Schaben (Blatta orientalis)

Der Boden eines 1 l-Einmachglases wird mit der acetonischen Lösung des Wirkstoffs behandelt. Nach Verdunsten des Lösungsmittels setzt man je Glas 5 adulte Schaben.

Die Mortalitätsrate wird nach 48 Stunden bestimmt.

| Wirkstoff Nr. | Wirkstoffmenge [mg] | Mortalitätsrate [%] |
|---|---|---|
| 1 | 0,1 | 100 |
| | 0,05 | 80 |
| 5 | 0,1 | 100 |
| | 0,05 | 70 |
| 8 | 0,1 | 100 |
| Vergleichsmittel | 0,1 | 20 |

Beispiel C

Fraß- und Kontaktwirkung auf Raupen der Kohlschabe (Plutella maculipennis)

Blätter von jungen Kohlpflanzen werden 3 Sekunden lang in die wäßrige Wirkstoffemulsion getaucht und nach kurzem Abtropfen auf einen angefeuchteten Filter in eine Petrischale gelegt. Das Blatt wird darauf mit 10 Raupen des 4. Stadiums belegt.

Nach 48 Stunden beurteilt man die Wirkung.

| Wirkstoff Nr. | Mortalitätsrate in % bei einer Wirkstoffkonzentration von 0,001 [Gew.%] | 0,0005 [Gew.%] |
|---|---|---|
| 5 | 100 | 80 |

Beispiel D

Kontaktwirkung auf Moskito-Larven (Aedes aegypti)

200 ml Leitungswasser werden mit der Wirkstoffaufbereitung versetzt und darauf mit 30 bis 40 Moskito-Larven im 4. Larvenstadium besetzt. Die Versuchstemperatur beträgt 20°C. Nach 24 Stunden wird die Wirkung ermittelt.

| Wirkstoff Nr. | Konzentration der Wirkstoffaufbereitung [ppm] | Mortalitätsrate [%] |
|---|---|---|
| 5 | 0,01 | 100 |
| 5 | 0,004 | ca. 80 |

Beispiel E

Kontaktwirkung auf Zecken (Ornithodorus moubata)

Geprüft wird mit Zecken im 3. Larvenstadium. Dazu taucht man die Tiere, die sich in einem Papierbeutel befinden, für 3 Sekunden in die Prüfemulsion. Die Beutel werden frei

aufgehängt. Nach 48 Stunden wird die Wirkung auf die Zecken beurteilt.

| Wirkstoff Nr. | Wirkstoffkonzentration der Prüfemulsion [Gew.%] | Mortalitätsrate [%] |
|---|---|---|
| 5 | 0,0001 | 100 |
| 5 | 0,00005 | 80 |

Patentansprüche

1. Carbonsäureester der Formel I

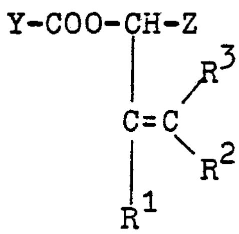

I,

in der
$R^1$, $R^2$ und $R^3$ gleich oder verschieden sind und für Wasserstoff, Halogen, Alkyl oder Alkenyl mit bis zu 5 Kohlenstoffatomen,
Y für einen 3-(2,2-Dihalogenvinyl)-2,2-dimethyl-cyclopropylrest oder einen Rest der Formel

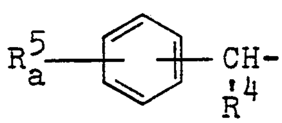

,

wobei $R^4$ unverzweigtes oder verzweigtes Alkyl, Alkenyl oder Alkinyl mit bis zu 4 Kohlenstoffatomen oder einen alicyclischen Rest mit 3 bis 7 Kohlenstoffatomen,
$R^5$ Halogen, Alkyl oder Alkoxy mit bis zu 5 Kohlenstoffatomen, Trihalogenmethyl, Cyano oder Nitro und
a 0 bis 3 bedeuten, und
Z für einen Rest der Formel

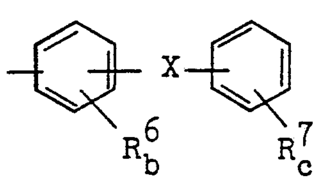

oder

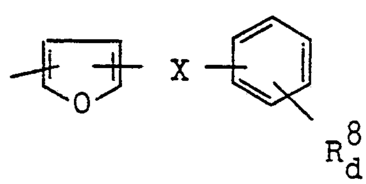

wobei X Sauerstoff, Schwefel oder $-CH_2-$, $R^6$, $R^7$ und $R^8$ Halogen, Alkyl, Alkoxy oder Halogenalkyl mit bis zu 5 Kohlenstoffatomen und b, c und d 0 bis 3 bedeuten, stehen.

2. Carbonsäureester der Formel I gemäß Anspruch 1, dadurch gekennzeichnet, daß $R^1$, $R^2$ und $R^3$ Wasserstoff bedeuten und die Substituenten Y und Z die im Anspruch 1 genannten Bedeutungen haben.

3. Carbonsäureester der Formel I gemäß Anspruch 1, dadurch gekennzeichnet, daß $R^1$, $R^2$ und $R^3$ Fluor bedeuten und die Substituenten Y und Z die im Anspruch 1 genannten Bedeutungen haben.

4. Carbonsäureester der Formel I gemäß Anspruch 1, dadurch gekennzeichnet, daß Z den 5-Benzylfuryl-3-rest bedeutet und $R^1$, $R^2$, $R^3$ und Y die im Anspruch 1 genannten Bedeutungen haben.

5. Schädlingsbekämpfungsmittel, enthaltend mindestens einen Carbonsäureester der Formel I gemäß Anspruch 1.

6. Verwendung von Carbonsäureestern der Formel I gemäß Anspruch 1 zur Bekämpfung von Schädlingen.

7. Verfahren zur Herstellung von Carbonsäureestern der Formel I gemäß Anspruch 1, dadurch gekennzeichnet, daß man Säurehalogenide der Formel II

Y-CO-Hal II,

in der
Y die in Anspruch 1 genannten Bedeutungen hat und
Hal für Halogen steht,

in Gegenwart eines säurebindenden Mittels mit Verbindungen der Formel III

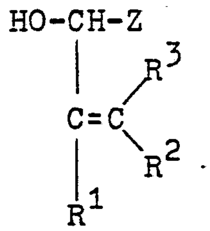

III,

in der
$R^1$, $R^2$, $R^3$ und Z die in Anspruch 1 genannten Bedeutungen haben,
umsetzt.

Patentansprüche Österreich

1. Schädlingsbekämpfungsmittel, enthaltend mindestens einen Carbonsäureester der Formel I

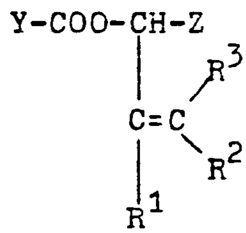

I,

in der
$R^1$, $R^2$ und $R^3$ gleich oder verschieden sind und für Wasserstoff, Halogen, Alkyl oder Alkenyl mit bis zu 5 Kohlenstoffatomen,
Y für einen 3-(2,2-Dihalogenvinyl)-2,2-dimethyl-cyclopropylrest oder einen Rest der Formel

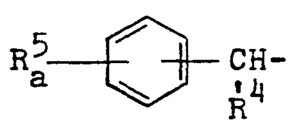

,

wobei $R^4$ unverzweigtes oder verzweigtes Alkyl, Alkenyl oder Alkinyl mit bis zu 4 Kohlenstoffatomen oder einen alicyclischen Rest mit 3 bis 7 Kohlenstoffatomen,
$R^5$ Halogen, Alkyl oder Alkoxy mit bis zu 5 Kohlenstoffatomen, Trihalogenmethyl, Cyano oder Nitro und
a 0 bis 3 bedeuten, und
Z für einen Rest der Formel

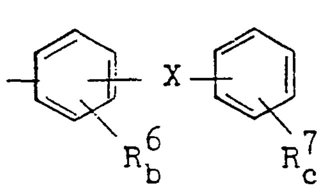

oder

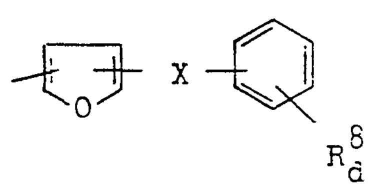

2. Verwendung von Carbonsäureestern der Formel I gemäß Anspruch 1 zur Bekämpfung von Schädlingen.

3. Verfahren zur Herstellung von Carbonsäureestern der Formel I gemäß Anspruch 1, dadurch gekennzeichnet, daß man Säurehalogenide der Formel II

Y-CO-Hal II,

in der
Y die in Anspruch 1 genannten Bedeutungen hat und
Hal für Halogen steht,
in Gegenwart eines säurebindenden Mittels mit Verbindungen der Formel III

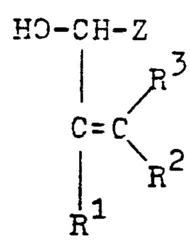

III,

in der
$R^1$, $R^2$, $R^3$ und Z die in Anspruch 1 genannten Bedeutungen haben,
umsetzt.

4. Schädlingsbekämpfungsmittel gemäß Anspruch 1, dadurch gekennzeichnet, daß $R^1$, $R^2$ und $R^3$ Wasserstoff bedeuten und die Substituenten Y und Z die in Anspruch 1 genannten Bedeutungen haben.

5. Schädlingsbekämpfungsmittel gemäß Anspruch 1, dadurch gekennzeichnet, daß $R^1$, $R^2$ und $R^3$ Fluor bedeuten und die Substituenten Y und Z die in Anspruch 1 genannten Bedeutungen haben.

6. Schädlingsbekämpfungsmittel gemäß Anspruch 1, dadurch gekennzeichnet, daß Z den 5-Benzylfuryl-3-rest bedeutet und $R^1$, $R^2$, $R^3$ und Y die in Anspruch 1 genannten Bedeutungen haben.

Europäisches Patentamt

# EUROPÄISCHER RECHERCHENBERICHT

0012273

Nummer der Anmeldung

EP 79 10 4736

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| PX | EP - A - 0 001 824 (CIBA-GEIGY) * Seite 1 bis Seite 2, Gleichung II; Seite 4, Absätze 2-5; Seite 9, Beispiel 1; Seite 13; Anspruch 5 * | 1,2,5-7 |
| A | DE - A - 2 750 844 (CIBA-GEIGY) * Seiten 1,2; Ansprüche 1,4-6 * | 1,5-7 |

KLASSIFIKATION DER ANMELDUNG (Int.Cl. 3)

C 07 C 69/747
69/767
67/14
A 01 N 53/00
C 07 D 307/28
A 01 N 43/08
37/10//
C 07 C 43/295
33/28

RECHERCHIERTE SACHGEBIETE (Int. Cl. 3)

C 07 C 69/747
67/14
69/767

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 10-03-1980 | KINZINGER |